# FASCICULE DE BREVET EUROPEEN

(11) **EP 1 786 862 B1**
(45) Date de publication et mention de la délivrance du brevet: **07.08.2019**
(21) Numéro de dépôt: 05717471.6
(22) Date de dépôt: 21.01.2005
(51) Int. Cl.: B82B 1/00, C08K 9/06, A61K 8/26, A61Q 19/00, A61K 8/02, A61K 33/06, A61K 35/02, A61K 36/00, C08K 9/08, A23K 10/30, A23L 29/00, A23L 29/294, A61K 8/9706, A23K 20/28

(54) **ARGILES INTERCALEES**
INTERKALIERTER TON
INTERSPERSED CLAY

(30) Priorité: 09.09.2004 FR 0409583
(43) Date de publication de la demande: 23.05.2007
(73) Titulaire: Olmix, 56580 Brehan (FR)
(72) Inventeur: DEMAIS, Hervé, F-56390 Brandivy (FR); BRENDLE, Jocelyne, F-68270 Wittenheim (FR); LE DEIT, Hervé, Servel F-22300 Lannion (FR); LAZA, Anca L., F-68350 Brunstatt (FR); LURTON, Luc, F-22610 Pleubian (FR); BRAULT, Dominique, F-22740 Lezardrieux (FR)
(74) Mandataire: Lavoix
(86) Numéro de dépôt international: PCT/FR2005/000145
(87) Numéro de publication internationale: WO 2006/030075

(56) Documents cités:
- WO-A1-96/25055
- FR-A1- 2 406 395
- US-A- 3 901 976
- US-A- 4 888 185
- "LA SOUPE EN ENGRAISSEMENT: DES AUGES PROPRES ET DES PORCS QUI POUSSENT" PORC MAGAZINE, PORC MAGAZINE, RENNES, FR, no. 363, février 2003 (2003-02), pages 134-137, XP001180333 ISSN: 0296-9076

## Description

La présente invention concerne des compositions à base d'argile et d'extraits d'algues.

Les argiles sont des roches composées principalement de silicates en feuillets (phyllosilicates) plus ou moins hydratés. Les phyllosilicates sont les composés de l'anion orthosilicate où les tétraèdres partagent entre eux trois de leurs oxygènes, le quatrième étant toujours tourné du même côté du feuillet ainsi formé. La structure peut être représentée comme un assemblage bidimensionnel de deux types de forme géométrique: l'octaèdre et le tétraèdre. Trois types de phyllosilicates sont ainsi définis:
- les phyllosilicates 1:1 dont le feuillet est constitué par la juxtaposition d'une couche tétraédrique à une couche octaédrique. L'épaisseur de ce type de feuillet est de 0,70 nm. La kaolinite est le composé le plus représentatif de ce groupe.
- les phyllosilicates 2:1 dont le feuillet est constitué d'une couche octaédrique comprise entre deux couches tétraédriques. L'épaisseur de ce type de feuillet est de 0,96 nm.
- les phyllosilicates 2:1:1 dont le feuillet est constitué d'une couche de brucite Mg(OH)₂ ou de gibbsite Al(OH)₃ dans l'espace interlamellaire. L'épaisseur de ce type de feuillet est de 1,4 nm.

Les phyllosilicates 2:1 présentent les propriétés les plus intéressantes du fait de leur structure. Les cavités de la couche tétraédrique d'un feuillet contiennent essentiellement des ions silicium et les cavités de la couche octaédrique des ions aluminium ou magnésium. Cependant de nombreuses substitutions peuvent avoir lieu dans les différentes couches. Les ions silicium sont substitués par des cations trivalents. Les ions aluminium ou magnésium sont substitués par des ions tri- ou divalents. Ces substitutions introduisent un excès de charge négative dans le feuillet. Elle est compensée par la présence de cations dans l'espace interfoliaire. Ces cations peuvent être échangés contre d'autres cations d'origine minérale ou organique. L'épaisseur de l'espace interfoliaire peut ainsi être modulé en fonction de l'application visée. Divers procédés de modification de la structure des phyllosilicates ont ainsi vu le jour :
- le procédé de pontage : traitement en deux étapes qui vise tout d'abord à remplacer les cations interfoliaires par des polycations (à base d'aluminium (Al₁₃O₄(OH)₂₄(H_{2O})₁₂]⁷⁺ (Diddams P.A, Thomas, J.M., Jones W., Ballantine J.A . et Purnell, J. (1984)., Chem. Soc. Chem. Commun, 106, 1340), de zirconium ((Zr₄(OH)₁₂(H₂0)₁₂⁴⁺, (Yamanaka S. et Brindley G.W. (1979) Clays and Clay Minerais, 27, 119.), puis à calciner le composé obtenu à une température suffisante pour transformer les polycations en particules de pseudo-oxydes. Les phyllosilicates pontés (encore appelés phyllosilicates à piliers) obtenus par cette procédure sont des solides poreux bidimensionnels possèdant un rayon de pore couvrant un large domaine (1,5 à 10,0 nm) et une activité catalytique importante liée à la fois aux sites acides des feuillets et des piliers.
- La transformation de phyllosilicates hydrophiles en phyllosilicates organophiles (A. Weiss (1963) Angew. Chem. Internat. Edit., 2, 134). Le traitement consiste à introduire des cations organiques (comme par exemple des ions alkylammoniums, de formule CH₃-(CH₂)ₙ-NH₃⁺, où n est compris entre 1 et 20) dans l'espace interfoliaire par échange d'ions. Dans ce cas, l'épaisseur de l'espace interfoliaire dépend à la fois de la nature du phyllosilicate, de la localisation de la charge et du nombre d'atomes de carbone présent dans la chaîne carbonée. Ces phyllosilicates organophiles deviennent compatibles avec des matrices polymères et sont employés comme charges dans les dits-polymères. Il existe à ce jour trois catégories de composites phyllosilicates-polymère (Alexandre, M. et Dubois P. (2000), Mater. Sci. En., 28, 1).
   - Les composites dits microcomposites, dans lequel le polymère ne pénètre pas dans l'espace interfoliaire du phyllosilicate. Ce dernier joue alors le rôle d'un renfort.
   - Les nanocomposites intercalés dans lesquels le polymère est inséré entre les feuillets.
   - Les nanocomposites exfoliés, où les feuillets de dimension nanométriques sont totalement dispersés dans la matrice polymère, formant ainsi une structure monolithique à l'échelle microscopique.

Les nanocomposites exfoliés présentent un intérêt tout particulier dans la mesure où les interactions phyllosilicates-polymère sont maximales, la totalité de la surface des feuillets étant disponible dans ce cas. Les propriétés mécaniques, de tenue au feu, la stabilité thermique et les propriétés barrières (perméabilité aux gaz, aux hydrocarbures,...) peuvent ainsi être améliorées.

Les principales voies d'obtention de nanocomposites sont les suivantes:
- Par polymérisation *in situ* (Okada, A., Kawasumi M., Usuki A., Kojima Y., Kurauchi T. and Kamigaito (1990) Mater. Res. Soc. Pro., 171, 45.) : procédé en deux étapes qui consiste tout d'abord à faire gonfler un phyllosilicate organophile dans une solution de monomère puis à y ajouter un agent de traitement afin d'initier la polymérisation. Lors du gonflement, les molécules de monomères polaires vont diffuser entre les feuillets. les ions alkylammonium présents dans l'espace interfoliaire vont alors s'orienter perpendiculairement aux feuillets pour optimiser les interactions avec le monomère. L'ajout de l'agent de traitement induit la polymérisation provoquant l'exfoliation du phyllosilicate.
- Par formation *in situ* de silicates en feuillets (Carrado K. and Xu L.Q ; (1998) Chem. Mater., 10, 1440-1445) : cette méthode récente consiste à réaliser une cristallisation hydrothermale in situ de silicates en feuillets comme l'hectorite dans un gel polymère aqueux.
- En solution : dans ce cas, le composé lamellaire organophile est exfolié en utilisant un solvant organique adéquat dans lequel le polymère est soluble. Le gain d'entropie obtenu par la désorption des molécules de solvants va alors permettre aux chaînes de polymère de diffuser entre les feuillets d'argile. Le solvant est ensuite évaporé.
- Par fusion (Vaia R.A. Ishii H. and Giannelis E.P. (1993) Chem. Mater, 5, 1694) : le phyllosilicate organophile est dans ce cas mélangé avec le polymère, L'ensemble est fondu puis recuit à une température supérieure à la température de transition vitreuse, processus conduisant à la formation du nanocomposite.

Parallèlement à ces procédés, une méthode par voie photochimique a également vu le jour (Koch T., Menning M. et Schmidt H. (1999) Adv. Sci. Technol., 17, 681., Zahouily K. , Benfahri S., Bendaikha T., Baron J. et Decker C. (2001) Proc. RadTech Europe, 583.). Elle consiste à irradier sous UV une formulation composée d'un monomère (par exemple acrylique), d'un photoamorceur de polymérisation, d'un diluant réactif et d'un phyllosilicate organophile. Un nanocomposite transparent et non coloré, présentant par ailleurs des propriétés physico-chimiques renforcées par rapport à celle de la résine seule a ainsi pu être préparé en utilisant une résine polyuréthane-acrylate. Ce procédé, réalisé à température ambiante et en l'absence de solvants présente l'avantage d'être à la fois facile à mettre en oeuvre et respectueux de l'environnement.

La formation d'hétérostructures poreuses : Une nouvelle voie d'obtention de composés comportant des entités siliciques dans l'espace interfoliaire de smectites (Galarneau, A., Barodawalla, A. et Pinnavaia, T.J. (1995) Nature, 174, 529.) est basé sur l'assemblage de silice autour de micelles d'un tensioactif au sein même de l'espace interfoliaire. Le mécanisme de formation proposé par les auteurs est analogue à celui conduisant à des solides mésoporeux de type MCM-41. L'avantage des composés ainsi formés est de posséder une taille de pores et une surface spécifique supérieure à celles revendiquées pour les composés à piliers. Le mode opératoire nécessite trois étapes :
- la première consiste à introduire un ion alkylammonium (par exemple l'ion hexadecyltriméthylammonium, noté C₁₆ TMA)) dans l'espace interfoliaire par échange d'ions.
- la seconde vise à incorporer à une amine primaire (telle la dodecylamine) et un précurseur de silice (tetraéthylorthosilicate, TEOS) la dernière consiste à libérer la porosité par calcination ou par extraction du tensioactif dans un solvant acidifié.

Dans le premier cas, l'oxydation de la matière organique induit la formation des protons nécessaires à la neutralité électrique de la charpente. Ces matériaux possèdent donc une acidité intrinsèque, caractère qui permet d'envisager de nombreuses applications catalytiques (EP 1044721), J. A. Martens, E. Benazzi, J. Brendlé, S. Lacombe and R. Le Dred, Stud. Surf. Sci. Catal., 2000, 130, 293.).

Dans le second cas, les groupes Si-OH sont préservés. Il est dès lors possible de fonctionnaliser ces composés par greffage (Mercier L et Pinnavaia T.J. (1998) Microporous and Mesoporous Materials 20, 101.)

En dehors de leurs propriétés d'échanges cationiques, les phyllosilicates sont également connus pour leurs capacités d'adsorption. Ils forment en effet facilement des complexes lamellaires par l'insertion de molécules d'eau ou organiques dans l'espace interfoliaire. Ce phénomène, appelé gonflement dépend de la charge du feuillet, de la localisation de celle-ci (couche tétraédrique ou octaédrique) et de la nature des cations de compensation. Les cations divalents comme Mg²⁺ et Ca²⁺ facilitent l'adsorption 'eau dans l'espace interfoliaire en formant des macro-cations. L'adsorption de molécules organiques peut apporter un caractère hydrophobe au phyllosilicate.

Enfin, certains phyllosilicates présentent des propriétés acides, l'acidité étant liée par exemple à la substitution des ions silicium de la couche tétraédrique par des ions aluminium. La première acidité, dite de Brönsted, provient soit de la présence de protons dans l'espace interfoliaire, soit de la dissociation des molécules d'eau d'hydratation entourant les cations de compensation. La seconde acidité, dite de Lewis, est moins répandue : elle est due à l'existence de défauts ou de lignes de ruptures dans la structure du feuillet.

L'acidité des phyllosilicates est à l'origine de leurs propriétés catalytiques. Ils peuvent subir un traitement préalable pour améliorer leur activité catalytique : soit par un traitement acide, comme dans le cas de la montmorillonite commerciale K10, soit par un échange de cations.

La montmorillonite est à l'heure actuelle le phyllosilicate 2 :1 le plus étudié et le plus employé. Elle contient essentiellement les éléments silicium, aluminium et magnésium. Elle fait partie du groupe des smectites et du sous -groupe dioctaédrique.

Sa formule structurale théorique est :

C⁺₄ₓ[(M³⁺₄₍₁₋ₓ₎M²⁺_{4x2)VI}(Si⁴⁺₈)_{IV}O₂₀(OH)₄]

(C⁺ : cations de compensation de l'espace interfoliaire , M³⁺ : cation trivalent tel que Al³⁺, Fe³⁺ , M²⁺ : cation divalent tel que Mg²⁺, Cu²⁺, CO²⁺, Zn²⁺, Fe²⁺, Ni²⁺, x : taux de substitution octaédrique)

En réalité une montmorillonite naturelle contient souvent des substitutions tétraédriques en plus des substitutions octaédriques :

C⁺_{(4x+8y)}[(M³⁺₄₍₁₋ₓ₎M²⁺_{4x 2})_{VI}(Si_{8(1-y)}M³⁺_{8y})_{IV}O₂₀(OH)₄].

(y : taux de substitution tétraédrique).

Sa synthèse en phase pure a récemment été décrite (M. Reinholdt, J. Miehé-Brendlé, L. Delmotte, A.-M. Flank, R. Cortès, M.-H. Tuilier, et R. Le Dred, Eur. J. Inorg. Chem., 2001, 11, 2831.)

La demande FR 2 406 395 décrit un adjuvant alimentaire comprenant notamment un mélange d'algues marines ainsi qu'un mélange d'extraits d'artichaut, d'argile verte et de sorbitol.

La présente invention a pour objet de proposer une argile intercalée comportant un extrait d'algue comprenant des polysaccharides solubles dans l'eau comme composé intercalant, dans laquelle l'argile est une argile en feuillets présentant des propriétés de gonflement. Elle a également pour objet de proposer un procédé de préparation d'argiles intercalées, destinées en particulier à la préparation de nanocomposites argile-polymère et d'aliments pour animaux.

Dans le cadre de l'invention, on entend par le terme « algue » couvrir toutes les espèces végétales marines comportant des polysaccharides solubles dans l'eau, et en particulier les algues de l'espèce *ulva* (ulves). Ces algues sont connues pour proliférer sur les côtes, notamment atlantiques et méditerranéennes, d'où leur surnom de « marée verte ». Elles constituent une matière première disponible et d'accès aisé dont on recherche la valorisation.

Par ailleurs, certains constituants, notamment des polysaccharides solubles dans l'eau, extraits de ces algues présentent un intérêt à titre de nutriment, et à titre de matière polymère dans la cosmétique et pharmaceutique.

Dans le cadre de l'invention, les extraits d'algues utilisées sont de préférence des extraits d'algue d'ulve. Les extraits d'algue comprennent de préférence des ulvanes, en particulier plus de 80% en poids. La structure chimique de ces polysaccharides ulvanes n'est pas encore complètement élucidée. Toutefois, on sait qu'ils sont composés d'unités de rhamnose, xylose, glucose, acide glucoronique et sulfate.

On entend par le terme « argile » un phyllosilicate d'origine naturelle ou synthétique de structure appropriée pour l'intercalation de composés. Selon l'invention, on utilise des argiles qui présentent une structure en feuillets telles que les montmorillonites, la beidellite, la saponite, les illites, la glauconite, les chlorites, la vermiculite, les argiles fibreuses. Selon l'invention, on utilisera une argile possédant des propriétés de gonflement (smectite) et en particulier la montmorillonite. Bien entendu, il peut être envisagé d'utiliser plusieurs argiles en mélange.

L'intercalation entre argile et extraits d'algues se fera de préférence en mélangeant en phase aqueuse une argile et l'extrait d'algue dans un rapport de poids (extrait sec) argile / extrait d'algue de 0,1 à 80, de préférence de 1 à 30, de préférence encore de 2 à 15.

Selon un autre aspect, l'invention vise un procédé de préparation d'une argile intercalée par un extrait d'algue comprenant des polysaccharides solubles dans l'eau, dans laquelle l'argile est une argile en feuillets présentant des propriétés de gonflement, comportant les étapes consistant à :
i) préparer un extrait aqueux d'algue ;
ii) mettre en contact ledit extrait avec une argile dans un solvant pendant entre 30 secondes et 72 heures; et
iii) isoler l'argile intercalée obtenue.

La durée d'agitation du mélange argile extraits d'algues est comprise entre 30 secondes et 72 heures, de manière préférée entre 1 minute et 36 heures et de manière encore plus préférée entre 2 minutes et 24 heures. A l'issue du mélange, la phase solide de la suspension est séparée, par exemple centrifugée. Le solide recueilli est alors lavé puis séché.

Les argiles intercalées de cette façon avec des extraits d'algues présentent un écartement entre feuillets qui peut aller jusqu'à 30 À. Cet écartement important les rend très intéressantes pour un grand nombre d'applications.

En particulier, elles peuvent agir en l'état comme adsorbant de composés volumineux difficiles à capter par d'autres matériaux. Ce type de composés inclut notamment certaines toxines, comme les mycotoxines. Ainsi, les argiles intercalées décrites peuvent être utilisées à titre de supplémentation dans l'alimentation animale ou humaine.

Ainsi, selon un autre aspect, l'invention vise l'utilisation de l'argile intercalée, notamment dans l'alimentation animale et humaine, la cosmétique, la pharmacie, la plasturgie, dans les revêtements de surface, les emballages alimentaires ou non.

Ces propriétés trouveront des applications en particulier en alimentation animale pour améliorer le rendement alimentaire avec une incorporation de l'argile intercalée de l'ordre de 0.01 à 1% en poids dans l'aliment.

Selon un autre aspect, l'invention vise donc des aliments pour animaux comportant de préférence 0,01 à 2% en poids, et en particulier entre 0,05 et 1% en poids d'argile intercalée telle que décrite ci-dessus.

L'écartement des feuillets permet également de rendre accessible l'espace interfoliaire pour d'autres fonctionnalisations telles que le greffage de radicaux activateurs de réactions chimiques ou biochimiques.

En effet, les produits issus de la présente invention ont un espace interfoliaire accessible, contrairement à ce qui peut être observé dans le cas de l'intercalation avec d'autres polymères (Intercalation de chitosan, extrait de carapaces de crustacés, dans une argile, M. Darder et al., Chem. Mater. 2003, 15, 3774-3780). Il est donc envisageable de faire entrer très facilement d'autres composés dans la structure ce qui ouvre la voie en particulier à la synthèse de nanocomposites respectueux de l'environnement utilisables dans de très nombreux domaines tels que l'alimentation animale et humaine, la cosmétique, la pharmacie, la plasturgie, les emballages alimentaires ou non, les revêtements de surface, etc...

L'invention vise donc selon un dernier aspect des nanocomposites comprenant l'argile intercalée décrite et un polymère naturel ou synthétique

L'invention sera décrite plus en détail au moyen des exemples qui suivent.

### EXEMPLES

### EXEMPLE 1

Préparation d'une montmorillonite-Na de taux de substitution tétraédrique égal à 0,4 (M. Reinholdt, J. Miehé-Brendlé, L. Delmotte, A.-M. Flank, R. Cortès, M.-H. Tuilier, et R. Le Dred, Eur. J. Inorg. Chem., 2001, 11, 2831)

Une montmorillonite de formule chimique :
Nao,4 [Al(_{1,6} Mg_{0,4}]Si₄O₁₀(OH_{1,8}F_{0,2}) est préparé de la manière suivante :
8,1 g d'une solution d'acide fluorhydrique (HF, Fluka) à 5 % dans l'eau sont ajoutés sous agitation magnétique à 685,86 g d'eau distillée placés dans un Becher en PTFE. 8,64 g d'acétate de magnésium (Mg(CH₃COO)₂, Aldrich), 1,74 g d'acétate de sodium (Na(CH₃COO, Fluka), 10,53 g de pseudo-bohmite (Al₂O₃, Condéa) et 24,3 g de silice (SiO₂ Aerosil 130, Degussa) sont successivement ajoutés au milieu réactionnel sous agitation. L'ensemble est mûri sous agitation à température ambiante durant 2 heures avant d'être transvasé dans un autoclave chemisé en PTFE et placé dans une étuve à 220 °C durant 72 heures. L'autoclave est ensuite refroidi à température ambiante et le produit de la réaction est filtré sur Büchner. Après trois lavages successifs à l'eau distillée, le produit est séché durant 24 heures à 60 °C.

### EXEMPLE 2

### Préparation d'ulvane

Procédé d'extraction des ulvanes (Lahaye M., Birnalendu R., Baumberger S., Quernener B. and Axelos M. (1996) Hydrobiologia, 326/327, 473).

L'ulve séchée et broyée (34,4g) en suspension dans de l'eau (500 mL) est portée à reflux pendant 1h. La suspension est centrifugée (10.24xg, 20 min) et l'insoluble est récupéré puis extrait à nouveau dans les mêmes conditions que précédemment. La suspension est centrifugée. Les deux surnageants des deux extractions sont réunis, filtrés puis l'ulvane en solution est précipitée à l'alcool à 95 °C. Le produit est ensuite séché.

### EXEMPLE 3

### Incorporation d'ulvanes dans l'espace interfoliaire de montmorillonite-Na de synthèse

1 g de montmorillonite-Na préparée selon l'exemple 1 est mis en suspension dans 100 mL d'eau distillée (Solution A). L'ensemble est placé sous agitation magnétique à température ambiante durant 24 heures. Parallèlement à ceci, 5 g d'ulvanes préparé selon l'exemple 2 sont dispersés dans 50 ml d'eau distillée sous agitation magnétique à température ambiante durant 24 heures (solution B). La solution A est ensuite mélangée à la solution B et l'ensemble est laissé sous agitation magnétique durant 24 heures à température ambiante. La suspension est alors centrifugée durant 10 minutes (à une vitesse de 20000 rotation par minute). Le solide recueilli est remis en suspension dans 20 mL d'eau distillé puis séparé par centrifugation. Ce lavage est répété deux fois. Le solide est ensuite séché à l'air durant 24 heures. Le produit ainsi formé contient 29 % de matière organique. L'espacement interfoliaire est de 3,8 nm.

## Revendications

1. Argile intercalée, comportant une argile et un extrait d'algue comportant des polysaccharides solubles dans l'eau à titre de composé intercalant, dans laquelle l'argile est une argile en feuillets possédant des propriétés de gonflement.

2. Argile selon la revendication 1, dans laquelle l'extrait d'algue est un extrait d'algue d'ulve.

3. Argile selon la revendication 1 ou 2, dans laquelle l'extrait d'algue comprend des ulvanes.

4. Argile selon l'une des revendications 1 à 3, dans laquelle l'extrait d'algue comporte plus de 80% en poids d'ulvanes.

5. Argile selon l'une des revendications 1 à 4, dans laquelle l'argile est une montmorrillonite.

6. Argile selon l'une des revendications 1 à 5, comportant l'argile et l'extrait d'algue dans un rapport en poids argile/extrait d'algue de 0,1 à 80, de préférence de 1 à 30 et de manière encore plus préférée de 2 à 15.

7. Procédé de préparation d'une argile intercalée par un extrait d'algue comportant des polysaccharides solubles dans l'eau, dans lequel l'argile est une argile en feuillets possédant des propriétés de gonflement, comportant les étapes consistant à :
i) préparer un extrait aqueux d'algue ;
ii) mettre en contact ledit extrait avec une argile dans un solvant pendant entre 30 secondes et 72 heures; et
iii) isoler l'argile intercalée obtenue.

8. Utilisation de l'argile intercalée selon l'une des revendications 1 à 6 dans l'alimentation animale et humaine, la cosmétique, la pharmacie, la plasturgie, dans les revêtements de surface, les emballages alimentaires ou non.

9. Aliments pour animaux comportant 0,01 à 2% en poids, de préférence entre 0,05 et 1% en poids d'argile intercalée selon l'une des revendications 1 à 6.

10. Nanocomposites, comprenant une argile intercalée selon l'une des revendications 1 à 6 et un polymère naturel ou synthétique.

## Patentansprüche

1. Interkalierter Ton, aufweisend Ton und ein Algenextrakt, welches wasserlösliche Polysaccharide als interkalierende Verbindung aufweist, wobei der Ton ein Schichtton ist, der Quelleigenschaften besitzt.

2. Ton gemäß Anspruch 1, wobei das Algenextrakt ein Ulva-Algenextrakt ist.

3. Ton gemäß Anspruch 1 oder 2, wobei das Algenextrakt Ulvane aufweist.

4. Ton gemäß einem der Ansprüche 1 bis 3, wobei das Algenextrakt mehr als 80 Gew.-% Ulvane aufweist.

5. Ton gemäß einem der Ansprüche 1 bis 4, wobei der Ton Montmorillonit ist.

6. Ton gemäß einem der Ansprüche 1 bis 5, aufweisend den Ton und das Algenextrakt in einem Gewichtsverhältnis Ton/Algenextrakt von 0,1 bis 80, vorzugsweise von 1 bis 30 und noch bevorzugter von 2 bis 15.

7. Verfahren zum Herstellen von interkaliertem Ton mittels eines Algenextrakts, welches wasserlösliche Polysaccharide aufweist, wobei der Ton ein Schichtton ist, der Quelleigenschaften besitzt, aufweisend die folgenden Schritte:
i) Herstellen eines Algenextrakts,
ii) in-Kontakt-Bringen des Extrakts mit einem Ton in einem Lösungsmittel während zwischen 30 Sekunden und 72 Stunden und
iii) Isolieren des erhaltenen interkalierten Tons.

8. Verwendung des interkalierten Tons gemäß einem der Ansprüche 1 bis 6 in Tiernahrung und Menschennahrung, Kosmetik, Pharmazie, Kunststoffindustrie, in Oberflächenbeschichtungen, Lebensmittel- oder Nicht-Lebensmittelverpackungen.

9. Nahrungsmittel für Tiere, aufweisend 0,01 bis 2 Gew.-%, vorzugsweise zwischen 0,05 und 1 Gew.-% interkalierten Ton gemäß einem der Ansprüche 1 bis 6.

10. Nanokomposite, die interkalierten Ton gemäß einem der Ansprüche 1 bis 6 und ein natürliches oder synthetisches Polymer aufweisen.

## Claims

1. Intercalated clay, comprising a clay and an algae extract comprising water-soluble polysaccharides as the intercalating compound, wherein the clay is a sheet clay having swelling properties.

2. Clay according to claim 1, wherein the algae extract is an Ulva algae extract.

3. Clay according to claim 1 or 2, wherein the algae extract comprises ulvans.

4. Clay according to any one of claims 1 to 3, wherein the algae extract comprises more than 80% by weight ulvans.

5. Clay according to any one of claims 1 to 4, wherein the clay is a montmorillonite.

6. Clay according to any one of claims 1 to 5, comprising the clay and the algae extract in a weight ratio of clay/algae extract of from 0.1 to 80, preferably from 1 to 30 and yet more preferably from 2 to 15.

7. Method for preparing a clay intercalated by an algae extract comprising water-soluble polysaccharides, wherein the clay is a sheet clay having swelling properties, comprising steps consisting in:
i) preparing an aqueous algae extract;
ii) bringing said extract into contact with a clay in a solvent for between 30 seconds and 72 hours; and
iii) isolating the intercalated clay obtained.

8. Use of the intercalated clay according to any one of claims 1 to 6 in animal and human food, cosmetics, pharmaceutics, plastics technology, in surface coatings, food or non-food packaging.

9. Animal feed comprising from 0.01 to 2% by weight, preferably between 0.05 and 1% by weight, intercalated clay according to any one of claims 1 to 6.

10. Nanocomposites, comprising an intercalated clay according to any one of claims 1 to 6 and a natural or synthetic polymer.
